(19) Europäisches Patentamt
European Patent Office
Office européen des brevets

(11) EP 1 488 788 A1

(12) EUROPEAN PATENT APPLICATION

(43) Date of publication:
22.12.2004 Bulletin 2004/52

(21) Application number: 04020247.5

(22) Date of filing: 08.12.2000

(51) Int Cl.[7]: A61K 31/4045, A61K 31/138, A61K 31/195, A61P 1/00, A61K 31/135
// (A61K31/4045, 31:138), (A61K31/4045, 31:195)

(84) Designated Contracting States:
AT BE CH CY DE DK ES FI FR GB GR IE IT LI LU MC NL PT SE TR
Designated Extension States:
RO SI

(30) Priority: 10.12.1999 US 458388

(62) Document number(s) of the earlier application(s) in accordance with Art. 76 EPC:
00989967.5 / 1 286 668

(71) Applicants:
- **Novartis AG**
  **4056 Basel (CH)**
  Designated Contracting States:
  **BE CH LI CY DE DK ES FI FR GB GR IE IT LU MC NL PT SE TR**
- **Novartis Pharma GmbH**
  **1230 Wien (AT)**
  Designated Contracting States:
  **AT**

(72) Inventors:
- **Billstein, Stephan Anthony**
  **Franklin Lakes NJ 07417 (US)**
- **Dumovic, Peter**
  **Mendham NJ 07945 (US)**
- **Franco, Nicola**
  **68480 Sondersdorf (FR)**
- **Iwicki, Mark Thomas**
  **Long Valley NJ 07853 (US)**
- **Pfannkuche, Hans-Jürgen**
  **79576 Weil (DE)**
- **Wilusz, Edward Joseph, Jr.**
  **Pittstown NJ 08867 (US)**

(74) Representative: **Grubb, Philip William et al**
**Novartis AG,**
**Corporate Intellectual Property**
**4002 Basel (CH)**

Remarks:
This application was filed on 26 - 08 - 2004 as a divisional application to the application mentioned under INID code 62.

# (54) Pharmaceutical combinations and their use in treating gastrointestinal disorders

(57) The present invention relates to a combination of a first agent including either a 5-$HT_4$ receptor agonist or antagonist or a 5-$HT_3$ receptor antagonist and a co-agent and pharmaceutical compositions and formulations containing the combination. The pharmaceutical combination may be employed for the treatment of altered gastrointestinal motility, sensitivity, secretion or abdominal disorders. The dosage is preferably oral. The preferred first agent is tegaserod.



Printed by Jouve, 75001 PARIS (FR)

## Description

The present invention relates to pharmaceutical combinations comprising at least one agent interacting with a $5\text{-}HT_3$ or $5\text{-}HT_4$ receptor and their uses in treating gastrointestinal disorders.

Serotonin (5-hydroxytryptamine; 5-HT) functions as a neurotransmitter in the mammalian central nervous system (CNS) and in the periphery. Serotonin is one of the transmitters to be recognized for its physiological importance, and agents which interact with 5-HT receptors are currently the focus of much research (P. Bonate, Clinical Neuropharmacology, 1991, Vol. 14(1), pp. 1-16). To-date, the number of serotonin receptor subtypes identified is into the tens, including the major classes, i.e., $5\text{-}HT_1$, $5\text{-}HT_2$, $5\text{-}HT_3$, $5\text{-}HT_4$, $5\text{-}HT_5$, $5\text{-}HT_6$, and $5\text{-}HT_7$. Because of the multiplicity of serotonin receptor subtypes, the identification of which serotonin receptor subtype is correlated to various physiological/ pharmacological actions is complicated.

Serotonin has been known for some years to modulate peristalsis in the gastro-intestinal (GI) tract in various mammalian models. During the mid 1980s, several specific antagonists to the $5\text{-}HT_3$ receptor subtype were identified and are currently used as anti-emesis/vomiting agents in cancer therapy. $5\text{-}HT_3$ antagonists have also recently been studied for the treatment of irritable bowel syndrome ("IBS").

A number of gastrointestinal syndromes are related to the production and actions of serotonin, and they have a fairly common occurrence in a very large number of people worldwide. Some of the more well-known gastrointestinal conditions, syndromes or diseases are IBS, gastro-esophageal reflux disease ("GERD") and dyspepsia.

IBS is a chronic condition associated with abdominal pain, bloating and altered bowel function and is estimated to affect as much as 10-20% of the population. Sometimes the disease is referred to as irritable colon, spastic colon, spastic colitis or mucous colitis. The latter two are almost certainly misnomers, as colitis implies inflammation of the colon, and an absence of inflammation is one of the defining observations in a diagnosis of IBS. The cause of IBS is unknown, but a number of factors have been implicated, including diet, lifestyle, depression, anxiety, infections and unrelated inflammatory conditions, including early insult resulting in central neuronal sensitization and sensitizing of neurons in the gut.

Almost all medications currently in use for the treatment of IBS have failed to establish a significant therapeutic effect.

GERD is a condition that is associated with the reflux of gastric contents to the esophagus through the lower esophageal sphincter. GERD is characterized by symptoms of heartbum, bloating, abdominal pain, epigastric pain, early satiety, nausea, regurgitation, burbulence and vomiting. The reflux is thought to occur because of an increased incidence of transient lower esophageal sphincter relaxations allowing gastric contents to enter the esophagus.

Dyspepsia is also an important health problem. The most common conditions that are associated with patients who present with chronic symptoms of dyspepsia are GERD, duodenal ulcer or gastric ulcer and other diagnoses (e. g. functional/non-ulcerative dyspepsia, gallbladder or liver disease).

These conditions or diseases are characterized by altered motility, sensitivity, secretion and/or infections with Helicobacter pylori as well as potentially a psychological (usually subconscious) overlay. At present, only few medications have shown clinically significant efficacy for the treatment of e.g. functional dyspepsia, of which some do exert various adverse effects in man.

Accordingly, there is a need for agents which modulate and normalize altered GI motility, sensitivity and secretion, and interact with 5-HT receptors involved in various physiological processes and which have broad clinical usefulness for the treatment of the numerous gastrointestinal disorders affecting millions of people each year. More specifically, there is a need for pharmaceutical combinations comprising either $5\text{-}HT_4$ receptor agonists or antagonists, $5\text{-}HT_4$ receptor partial agonists or $5\text{-}HT_3$ receptor antagonists and a co-agent effective for the treatment of altered gastrointestinal motility, sensitivity and/or secretion and/or abdominal disorders including both functional and organic diseases.

It has now been found that a combination comprising at least one agent interacting with a $5\text{-}HT_3$ or a $5\text{-}HT_4$ receptor, e.g., as defined below, and a co-agent, e.g., as defined below, has a beneficial effect and is useful in the treatment of altered gastrointestinal motility, sensitivity and/or secretion and/or abdominal disorders. This combination may also be used to regulate, stabilize and normalize altered gastrointestinal motility, sensitivity and/or secretion and/ or abdominal disorders.

### Definitions

Unless otherwise specified herein, common definitions are intended by the words and terms used herein. For example, the term "pharmaceutical combination" as used herein means a product that results from the mixing or combining of more than one active ingredient and includes both fixed and non-fixed combinations of the active ingredients.

The term "fixed combination" as that term is used herein means that the active ingredients, e.g. tegaserod and a co-agent, are both administered to a patient simultaneously in the form of a single entity or dosage. As an

example, a fixed combination would be one capsule containing two active ingredients.

The term "non-fixed combination" as that term is used herein means that the active ingredients, e.g. tegaserod and a co-agent, are both administered to a patient as separate entities either simultaneously, concurrently or sequentially with no specific time limits, wherein such administration provides therapeutically effective levels of the two compounds in the body, preferably at the same time. As an example, a non-fixed combination would be two capsules each containing one active ingredient where the purpose is to have the patient achieve treatment with both active ingredients together in the body.

The term "altered gastrointestinal motility, sensitivity and/or secretion disorder(s)" as used herein includes one or more of the symptoms and conditions which affect the gastrointestinal tract from the mouth to the anus, which include, but are not limited to, heartbum, bloating, postoperative ileus, abdominal pain and discomfort, early satiety, epigastric pain, nausea, vomiting, burbulence, regurgitation, intestinal pseudoobstruction, anal incontinence, GERD, IBS, dyspepsia, chronic constipation or diarrhea, gastroparesis, e.g. diabetic gastroparesis, ulcerative colitis, Crohn's disease, ulcers and the visceral pain associated therewith.

The term "abdominal disorder(s)" as used herein includes those conditions which affect the lower abdomen and include but are not limited to those conditions treated by regulation, stabilization and normalization of enterochromaffin cell functions, GI secretion, motility, afferent and efferent fiber activity and/or abdominal smooth muscle cell activity.

The term "gastro-esophageal reflux disease" and "GERD" as used herein means the incidence of, and the symptoms of, those conditions caused by the reflux of the stomach contents into the esophagus. This includes all forms/manifestations of GERD including, but not limited to, erosive and non-erosive GERD, heartbum and other symptoms associated with GERD.

The term "irritable bowel syndrome" and "IBS" as used herein means a disorder of function involving altered motility, sensitivity and secretion involving primarily the small intestine and large bowel associated with variable degrees of abdominal pain, bloating, constipation or diarrhea without overt bowel inflammation.

The term "dyspepsia" as used herein means a condition characterized by symptoms of epigastric pain, abdominal pain, bloating, early satiety, nausea, heartburn and vomiting as a primary gastrointestinal dysfunction or as a complication due, and not exclusive to disorders such as ulcer disease, appendicitis, gallbladder disturbances, or malnutrition.

The term "gastroparesis" as used herein means a paralysis of the stomach brought about by a motor abnormality in the stomach which is often manifested as delayed gastric emptying. This can also be a complication of diseases such as diabetes, progressive systemic sclerosis, anorexia nervosa, or myotonic dystrophy.

The term "constipation" as used herein means a condition characterized by infrequent and/or difficult evacuation of feces resulting from conditions such as altered GI motility, altered sensation or evacuation functions, altered secretion or reabsorption of electrolytes and water.

The term "diarrhea" as used herein means a condition characterized by frequent evacuations of feces often associated with large volumes and urge resulting from conditions such as altered GI motility, altered sensation and secretion or reabsorption of electrolytes and water.

The term "treat" or "treatment" encompasses the complete range of therapeutically positive effects associated with pharmaceutical medication including reduction of, alleviation of and relief from the symptoms or illness which affect the organism.

DESCRIPTION

More particularly the present invention provides:

1.1 A pharmaceutical combination comprising:

a) a first agent which is a 5-$HT_4$ receptor partial agonist; and
b) a co-agent.

5-$HT_4$ receptor partial agonists as a first agent in combination 1.1 include any compound which can partially activate 5-$HT_4$ receptors (intrinsic activity less than that of serotonin, i.e. < 1.00. The intrinsic activity may be determined in the non-electrically or electrically stimulated guinea pig ileum or striatum assay, e.g. as disclosed in EP-A1-0 505 322, Br. J. Pharmacol., 115, 1387, 1995 or in the guinea pig distal colon test e.g. as disclosed in Br. J. Pharm., 1593-1599, 1993).

In another embodiment, the invention provides:

1.2 A pharmaceutical combination comprising:

a) a first agent which is a compound of formula I

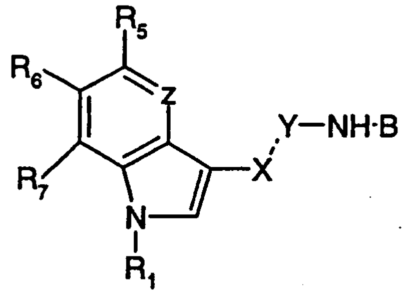

I

wherein

| | |
|---|---|
| $R_1$ | is hydrogen; $C_{1-6}$alkyl; ($C_{1-6}$alkyl)carbonyl; benzoyl; or phenyl$C_{1-4}$alkyl-carbonyl; |
| $R_5$ | is hydrogen; halogen; $C_{1-6}$alkyl; hydroxy; nitro; amino; $C_{1-6}$alkylamino; $C_{1-10}$alkylcarbonylamino; $C_{2-6}$alkoxycarbonyl; $SO_2NR_aR_b$ wherein each of $R_a$ and $R_b$ independently is hydrogen or $C_{1-6}$alkyl; cyano; or trimethylsilyl; $C_{1-6}$alkyl substituted by - $SO_2$-$C_{1-6}$alkyl, -$SO_2NR_aR_b$, -$CONR_aR_b$, -NH-$SO_2$-$C_{1-6}$alkyl, -N($C_{1-6}$alkyl)-$SO_2$-($C_{1-6}$alkyl), - $NR_aR'_b$ wherein $R'_b$ is hydrogen or $C_{1-6}$alkyl, $C_{2-6}$alkoxycarbonyl or -PO($C_{1-4}$alkyl)$_2$; carboxy; $CONR_aR_b$; -PO($C_{1-6}$alkyl)$_2$; $OCONR_cR_d$, wherein each of $R_c$ and $R_d$ independently is $C_{1-6}$alkyl; |
| $R_6$ | is hydrogen or, when $R_5$ is OH, $R_6$ is hydrogen or halogen, |
| Z | is -$CR_4$= wherein $R_4$ is hydrogen, halogen, hydroxy or $C_{1-6}$alkyl or, when $R_5$ is hydrogen or hydroxy, Z is also —N=, |
| $R_7$ | is hydrogen, halogen, $C_{1-6}$alkyl or $C_{1-6}$alkoxy, |
| X—Y | is —$CR_8$=N- or —CH($R_8$)-NH- wherein $R_8$ is hydrogen or $C_{1-6}$alkyl, and |
| B | is a radical of formula (a) or (b), |

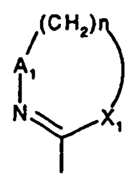

(a)

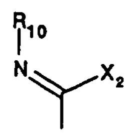

(b)

wherein

| | |
|---|---|
| n | is 1 or 2, |
| $A_1$ | is C=O or $CH_2$, |
| $X_1$ | is S; $NR_{11}$ wherein $R_{11}$ is hydrogen, ($C_{1-6}$alkyl)carbonyl, benzoyl or phenyl$C_{1-4}$alkyl-carbonyl; or $CR_{12}R_{13}$ wherein each of $R_{12}$ and $R_{13}$ independently is hydrogen or $C_{1-4}$alkyl, |
| $R_{10}$ | is hydrogen; $C_{1-12}$alkyl; $C_{1-6}$alkyl substituted by hydroxy, aryl, aryloxy, adamantyl, a heterocyclic radical, -$NR_{15}$-CO-$R_{16}$ or -NH-$SO_2$-aryl; $C_{5-7}$cycloalkyl; adamantyl; ($C_{1-10}$alkyl)carbonyl; benzoyl; phenyl ($C_{1-4}$alkyl)carbonyl; or —$CONHR_{14}$, |

wherein

| | |
|---|---|
| $R_{14}$ | is $C_{1-10}$alkyl or $C_{5-7}$cycloalkyl, |
| $R_{15}$ | is hydrogen or $C_{1-4}$alkyl, and |
| $R_{16}$ | is $C_{1-6}$alkyl, $C_{5-7}$cycloalkyl, $C_{5-7}$cycloalkyl-$C_{1-4}$alkyl, aryl or aryl$C_{1-4}$alkyl, |

wherever "aryl" appears as is or in the significances "aryloxy", "-NH-$SO_2$-aryl" or "aryl($C_{1-4}$alkyl)" in the above definition, it is phenyl or phenyl substituted by halogen, $C_{1-4}$alkyl or $C_{1-6}$alkoxy; and
wherever "heterocyclic radical" appears in the above definition, it is pyridyl, imidazolyl, benzimidazolyl, pyrrolidinyl, pyrrolidonyl, piperidino, pyrazinyl, perhydroindolyl or a radical of formula (c), (d) or (e)

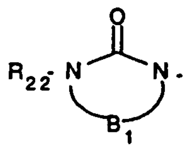

(c)

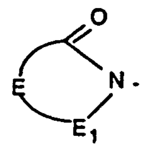

(d)

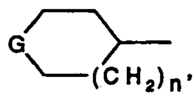

(e)

wherein

- $R_{22}$ is hydrogen or $C_{1-4}$alkyl,
- $B_1$ is $—CH_2CH_2$-, -$COCH_2$- or $—(CH_2)_3$- in which one or two H thereof can by replaced by $C_{1-4}$alkyl, or 1,2-phenylene,
- E is $—CH_2$-$CH_2$-, -$CH_2N(R_{17})$- or $—(CH_2)_3$- in which one or two H thereof can be replaced by $C_{1-6}$alkyl, or 1,2-phenylene,
- $E_1$ is CO or $CH_2$,
- $R_{17}$ is hydrogen or $C_{1-4}$alkyl,
- G is CO, -$CHCOOR_{18}$, -$CHCOR_{19}$, 5,5-dimethyl-1,3-dioxan-2-ylidene or 1,3-dioxolan-2-ylidene, wherein $R_{18}$ is hydrogen or $C_{1-6}$alkyl and $R_{19}$ is $C_{1-6}$alkyl, and
- n' is 0 or 1, and
- $X_2$ is $—SR_{20}$ or $—NR_3R'_{10}$ wherein $R_{20}$ is $C_{1-6}$alkyl, $R_3$ is hydrogen or $C_{1-6}$alkyl and $R'_{10}$ has one of the significances given for $R_{10}$ above, or $R_3$ and $R'_{10}$ together with the nitrogen atom to which they are attached form a heterocyclic radical as defined above;

with the proviso that where B is a radical of formula (b), only one of $R_{10}$ and $R'_{10}$ can be other than hydrogen and $X_2$ can be $—SR_{20}$ only when $R_{10}$ is hydrogen, and a physiologically-hydrolysable and -acceptable ether or ester thereof when $R_5$ is hydroxy,
in free form or in salt form, preferably in pharmaceutically acceptable salt form, and
b) a co-agent.

Compounds of formula I and their physiologically-hydrolysable and -acceptable ethers or esters are e.g. as disclosed in EP-A1-0 505 322. Suitable pharmaceutically acceptable salts are e.g. pharmaceutically acceptable acid addition salts, for example such salts as obtained with an inorganic or organic acid, e.g. the hydrochloride, sulfate, acetate, oxalate, maleate and fumarate salts.

By the term "physiologically-hydrolysable and -acceptable ethers or esters" as applied to the compounds of formula I when $R_5$ is hydroxy, is meant ethers in which $R_5$ is etherified (e.g. by optionally substituted $C_{1-6}$alkyl) and esters in which $R_5$ is esterified and which are hydrolysable under physiological conditions to yield an alcohol or acid which is physiologically acceptable, i.e. which is non-toxic at the desired dosage levels. Specific examples are given in EP-A1-0 505 322.

Representative 5-$HT_4$ receptor partial agonists include, but are not limited to, compounds of formula I having

an intrinsic activity less than that of serotonin. Preferred compounds of formula I as 5-$HT_4$ receptor partial agonists are e.g. those wherein $R_1$ is H, Z is -CH= and $R_5$ is OH or $C_{1-6}$alkoxy.

Further examples of 5-$HT_4$ receptor partial agonists include e.g. RS 67333 (1-(4-amino-5-chloro-2-methoxyphenyl)-3-[1-butyl-4-piperidinyl]-1-propanone), or RS 67506 (1-(4-amino-5-chloro-2-methoxyphenyl)-3-[1-methylsulphonylamino)ethyl-4-piperldinyl]-1-propanone).

Compounds of formula I above are preferred; a particularly preferred compound of formula I is the compound of formula

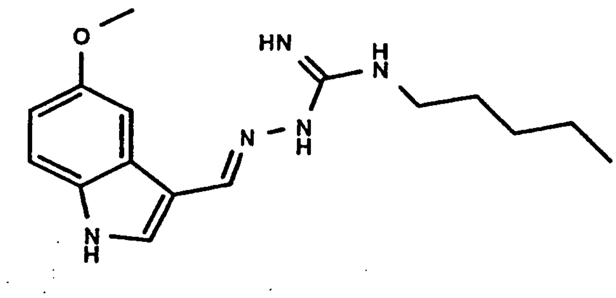

in free form or in pharmaceutically acceptable salt form. This compound has the chemical name of 3-(5-methoxy-1H-indol-3-yl-methylene)-N-pentylcarbazimidamide, and is also known as tegaserod. It is disclosed as being a 5-$HT_4$ receptor partial agonist. It may also exist in form of tautomers

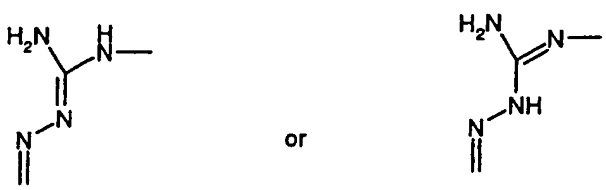

or

which are included in the present invention. A preferred salt form is the hydrogen maleate.

The co-agent in combination 1.1 or 1.2 is selected from the following groups of compounds: 5-$HT_3$ receptor antagonists, 5-$HT_4$ receptor agonists or antagonists, compounds which show characteristics of 5-$HT_3$ receptor antagonists and 5-$HT_4$ receptor agonists or antagonists, somatostatin receptor agonists, histamine $H_2$ receptor antagonists, proton pump inhibitors ("PPIs") which include irreversible, reversible and pro-drugs of PPIs, anxiolytics, e.g. chlordiazepoxide, benzodiazepine compounds, anti-spasmodic/anti-muscarinic agents, selective serotonin reuptake inhibitors ("SSRIs"), tricyclic antidepressants, selegeline, muscarinic$_1$ ("$M_1$") receptor agonists or antagonists, cholecystokinin ("CCK") receptor antagonists, opioid receptor agonists or antagonists, motilin receptor agonists or antagonists, nitric oxide synthase inhibitors, $GABA_B$ receptor agonists or modulators, neurokinin ("NK") receptor agonists or antagonists, calcitonin gene-related peptide receptor ("CGRP") or corticotropin releasing factor ("CRF") receptor agonists or antagonists, anti-inflammatory compounds, stimulant laxatives, osmotic laxatives, fecal softeners, absorbents and fiber supplements, antacids, GI relaxants, diphenoxylate, anti-gas compounds, bismuth-containing preparations, pentosan polysulfate, hydroxyzine, mast cell stabilizers and anti-emetic dopamine $D_2$ antagonists.

In an alternative embodiment, the invention further provides:

1.3. A pharmaceutical combination comprising

a) a first agent which is selected from the group consisting of 5-$HT_4$ receptor agonists or antagonists and 5-$HT_3$ receptor antagonists; and

b) a co-agent which is selected from the group consisting of somatostatin receptor agonists, anxyolitics, e.g. chlordiazepoxide, benzodiazepine compounds, anti-spasmodic/anti-muscarinic agents, tricyclic antidepressants, selegeline, $M_1$ receptor agonists or antagonists, CCK receptor antagonists, opioid receptor agonists or antagonists, motilin receptor agonists or antagonists, nitric oxide synthase inhibitors, $GABA_B$ receptor agonists or modulators, NK receptor agonists or antagonists, CGRP or CRF receptor agonists or antagonists, anti-inflammatory compounds, stimulant laxatives, osmotic laxatives, fecal softeners, absorbents and fiber supplements, antacids, GI relaxants, diphenoxylate, anti-gas compounds, bismuth-containing preparations, pentosan polysulfate, hydroxyzine and mast cell stabilizers,

provided that when agent a) is a 5-$HT_3$ receptor antagonist, co-agent b) is other than an antispasmodic/antimuscarinic agent, a $M_1$ receptor antagonist, an opioid receptor agonist or antagonist, a NK receptor antagonist, a stimulant laxative, an osmotic laxative or an anti-inflammatory corticosteroid.

In a further embodiment, the invention also provides:

1.4. A pharmaceutical combination comprising

a) a first agent which is a 5-$HT_4$ receptor antagonist; and
b) a co-agent which is selected from the group consisting of somatostatin receptor agonists, histamine $H_2$ receptor antagonists, PPIs, metoclopramide and anti-emetic dopamine $D_2$ antagonists.

The 5-$HT_4$ receptor agonists as a co-agent in combination 1.1 or 1.2 or as a first agent in combination 1.3 include any compound which can activate 5-$HT_4$ receptors under quiescent/resting conditions. These compounds include, but are not limited to, compounds of formula I as disclosed in EP- B1-0 505 322, cisapride, nor-cisapride, renzapride, zacopride, mosapride, prucalopride, SB 205149, SC 53116, RS 67333, RS 67506, BIMU-1, BIMU-8 and (S) -RS 56532. Cisapride, cis-4-amino-5-chloro-N-[1-[3-(4-fluorphenoxy)propyl]-3-methoxy-4-piperidinyl]-2-methoxy-benzamide, is in use as a gastro-prokinetic agent (See A. Reyntjens et al., Drug Div. Res., 8, 251 (1986) and Curr. Ther. Res., 36, 1029-1070 (1984)). The compound is marketed internationally under trade names such as ACENALIN®, PREPULSID®, RISAMOL®, PULSAR® and PROPULSIN®.

A preferred group of 5-$HT_4$ receptor agonists or partial agonists are those which are selective; by selective is meant a compound which does not substantially bind to or stimulate the 5-$HT_3$ receptor subtype. Tegaserod, for example, does neither bind to nor stimulate the 5-$HT_3$ receptor subtype.

5-$HT_4$ receptor antagonists for use as a co-agent in combination 1.1 or 1.2 or as a first agent in combination 1.3 or 1.4 include any compounds which bind to the 5-$HT_4$ receptor as defined by the IUPHAR (Pharmacological Reviews, Vol. 44, p. 157-213,1994) and that do not activate the 5-$HT_4$ receptor and antagonize the effects of serotonin. A relevant test to determine whether or not a compound is a 5-$HT_4$ receptor antagonist is the Guinea-Pig distal colon test as described in Br. J. Pharm., p. 1593-1599 (1993) or in the test described in Arch. Pharmacol., Vol. 343, p. 439-446 (1991). Representative 5-$HT_4$ receptor antagonists include e.g. piboserod; A-85380 (Abbott Laboratories) (WO 94/08994); SB 204070 (SmithKline Beecham) (Drugs Fut., 19:1109-1121, 1994) ; SB 207058 (Exp. Opin. Invest. Drugs, 3(7):767, 1994); SB 207710 (Drug Data Report, 15(10):949, 1993); SB 205800 (Drug Data Report, 15(10):949, 1993); SB 203186 (Br. J. Pharmacol., 110:1023-1030,1993); N 3389 (Nisshin Flour Milling) (Eur. J. Pharmacol., 271:159, 1994); FK 1052 (Fujisawa) (J. Pharmacol. Exp. Ther., 265:752, 1993); SC 56184 (Searle) (R&D Focus, 2(37) 10, 1993); SC 53606 (Searle/Monsanto) (J. Pharmacol. Exp. Ther. 226:1339,1993); DAU 6285 (Boerhinger Ingelheim) (Br. J. Pharmacol., 105:973,1992); GR 125487 (Glaxo) (Br. J. Pharmacol., 113 suppl. 119P & 120P, 1994); GR 113808 (Br. J. Pharmacol. 110:1172, 1993); RS 23597 (Syntex) (Bioorg Med. Chem. Lett., 4(20):2477, 1994); RS 39604 (Br. J. Pharmacol., 115, 1087-1095, 1995); LY0353433 (Eli Lilly Co. Ltd.) (J. Pharmacol. Exp. Ther., 277(1), 97-104, 1996); and R59595 (Eur. J. Pharmacol., 212, 51-59, 1992).

5-$HT_3$ receptor antagonists as a co-agent in combination 1.1 or 1.2 or as a first agent in combination 1.3 include compounds which bind to the 5-$HT_3$ receptor and antagonize the effect of a 5-$HT_3$ receptor agonist, e.g. cilansetron which is described in EP 29761; alosetron which is described in WO 99/17755; ramosetron; azasetron; ondansetron; dolasetron; ramosetron; granisetron; and tropisetron.

Compounds which show characteristics of 5-$HT_3$ receptor antagonists and 5-$HT_4$ receptor agonists or antagonists for use as a co-agent in combination 1.1 or 1.2 or as first agent in combination 1.3 are e.g. cisapride and nor-cisapride; BIMU compounds, for example BIMU1, BIMU8 and DAU 6215 (also known as itasetron) as disclosed in Dumuis A., et al., Naunyn Schmiedeber's Arch. Pharmacol., Vol. 343(3), pp. 245-251 (1991); DAU-6236 as disclosed in Rizzi, C.A. et al., J. Pharmacol. Exp. Ther., Vol. 261, pp. 412-419 (1992); and DAU-6258, Turconi M, et al., J. Med. Chem., Vol. 33(8), pg. 2101-2108 (1990), SDZ 205-557 which is a benzoic acid derivative (ester) Eglen R. M. et al., Proc. Br. Pharmacol. Soc., Vol. 149 (1992); renzapride; zacopride; SB 205149; SC 53116; RS 67333; RS 67506; or (S)-RS 56532, lintopride.

Examples of further co-agents which are combinable with the first agent according to the invention include, but are not limited to, the following:

i) Histamine $H_2$ receptor antagonists which include compounds that inhibit the action of histamine at the histamine $H_2$ receptors on gastric cells, e.g. famotidine marketed under the trade name PEPCID®; cimetidine marketed

under the trade name TAGAMET®; ranitidine marketed under the trade name ZANTAC®; and nizatidine marketed under the trade name AXID®.

ii) Irreversible Proton Pump Inhibitors (PPIs) which include compounds which inhibit gastric acid secretion by inhibition of the $H^+/K^+$ ATPase enzyme system of the gastric parietal cells, e.g. omeprazole marketed under the trade name PRILOSEC® and LOSEC®; lansoprazole marketed under the trade name PREVACID®; rabeprazole marketed under the trade name PARIET® and ACIPHEX®; pantroprazole marketed under the trade name PROTIUM®; and esomeprazole marketed under the trademark NEXIUM®. Reversible PPIs include, for example, BY 841, SKF 97574, SKF 96067, H 40502 and those disclosed in WO 98/43968 which are YH1238 and YH1885, Kim H. et al., Korean Journal of Physiology and Pharmacology, 1997, Vol 1(3), pp. 337-343.

iii) Benzodiazepine compounds and analogs which act to suppress seizures through an interaction with γ-aminobutyric acid (GABA) receptors of the A-type ($GABA_A$), for example, DIASTAT® and VALIUM®; LIBRIUM®; and ZANAX®.

iv) Anti-spasmodic/anti-muscarinic agents, for example, dicyclomine marketed under the trade name BENTYL®; hyoscyamine marketed under the trade name LEVSIN®; and darifenacin, (S)-1-[2-(2,3-Dihydro-5-benzofuranyl) ethyl]-α,α-diphenyl-3-pyrrolidineacetamide, described in U. S. Patent No. 5,837,724 which is a selective muscarinic $M_3$ receptor antagonist.

v) SSRIs, for example, fluvoxamine; fluoxetine; paroxetine; sertraline; citalopram; venlafaxine; cericlamine; duloxetine; milnacipran; nefazodone; and cyanodothiepin (See The Year Drugs News, 1995 Edition, pp. 47-48 by Prous J.R.) and WO 97/29739.

vi) Tricyclic anti-depressants, for example, amitriptyline marketed under the trade names ELAVIL®, ETRAFON®, LIMBITROL®, and TRIAVIL®; buproprion; and Sinequan.

vii) Selegeline, e.g. as marketed under the trade names ELDEPRYL®, ATAPRYL® and DEPRENYL®.

viii) CCK receptor antagonists, for example, devazepide; lorglumide; dexloxiglumide; loxiglumide, D'Amato, M. et al., Br. J. Pharmacol. Vol. 102(2), pp. 391-395 (1991 ); CI 988; L364,718; L363,260; L740,093 and LY288,513; CCK receptor antagonists disclosed in U. S. Patent No. 5,220,017, Bruley-Des-Varannes, S, et al. Gastroenterol. Clin. Biol. Vol. 15:(10), pp. 744-757 (1991), and Worker C: EUPHAR '99- Second European Congress of Pharmacology (Part IV) Budapest, Hungary Iddb Meeting Report 1999 July 3-7.

ix) Opioid receptor agonists or antagonists may be e.g. ADL8-2698, fedotozine, dextromethorphan, loperamide (e.g. IMODIUM®), diphenoxylate (e.g. LOMOTIL®), belladonna alkaloids, endorphin/enkephalin analogs.

x) Motilin receptor agonists or antagonists which include e.g. motilin agonist ABT-269, (erythromycin, 8,9-didehydro-N-dimethyl-9-deoxo-4",6,12-trideoxy-6,9-epoxy-N-ethyl), de(N-methyl-N-ethyl-8,9-anhydroerythromycin A) and de(N-methyl)-N-isoprop-8,9-anhydroerythromycin A), Sunazika T. et al., Chem. Pharm. Bull., Vol. 37(10), pp. 2687-2700 (1989); A-173508 (Abbot Laboratories); motilin antagonists (Phe3, Leu13) porcine motilin, $214^{th}$ American Chemical Society (ACS) Meeting (Part V); Highlights from Medicinal Chemistry Poster Session, Wednesday 10 September, Las Vegas, Nevada, (1997), Iddb Meeting Report September 7-11 (1997); and ANQ-11125, Peeters T.L., et al., Biochem. Biophys. Res. Commun., Vol. 198(2), pp. 411-416 (1994).

xi) $GABA_B$ receptor agonists or modulators, for example, (±)-baclofen, S(-)-baclofen, R(+)-baclofen, CGP44532, CGP47656, CGP7930, SK&F97541.

xii) NK receptor antagonists which include e.g. FK 888( Fujisawa); GR 205171 (Glaxo Wellcome); LY 303870 (Lilly); MK 869 (Merck); GR82334 (Glaxo Wellcome); L758298 (Merck); L 733060 (Merck); L 741671 (Merck); L 742694 (Merck); PD 154075 (Parke-Davis); S18523 (Servier); S19752 (Servier); OT 7100 (Otsuka); WIN 51708 (Sterling Winthrop); NKP-608A; TKA457; DNK333; CP-96345; CP-99994; CP122721; L-733060; L-741671; L-742694; L-758298; L-754030; GR-203040; GR-205171; RP-67580; RPR-100893 (dapitant); RPR-107880; RPR-111905; FK-888; SDZ-NKT-343; MEN-10930; MEN-11149; S-18523; S-19752; PD-154075 (CAM-4261); SR-140333; LY-303870 (lanepitant); EP-00652218; EP-00585913; L-737488; CGP-49823; WIN-51708; SR-48968 (saredutant); SR-144190; YM-383336; ZD-7944; MEN-10627; GR-159897; RPR-106145; PD-147714 (CAM-2291); ZM-253270; FK-224; MDL-105212A; MDL-105172A; L-743986; L-743986 analogs; S-16474; SR-142801

(osanetant); PD-161182; SB-223412; and SB-222200.

xiii) Calcitonin gene-related peptide (CGRP) receptor agonists or antagonists, which include e.g. CGRP-(8-37), Onodera S, et al., Jpn. J. Pharmacol., Vol. 68(2), pg. 161 -173 (1995) and Daines R. A. et al., Bioorganic Med. Chem. Lett., Vol. 7(20), pg. 2673-2676 (1997).

xiv) CRF receptor agonists or antagonists, e.g. as disclosed in WO 99/40089, AXC 2219, Antalarmin, NGD-98-1, CRA 0165, CRA 1000, CRA 1001.

xv) Somatostatin receptor agonists, e.g. octreotide, vapreotide, lanreotide.

xvi) Anti-inflammatory compounds, particularly those of the immuno-modulatory type, for example, NSAIDS; Tumor Necrosis Factor (TNF, TNF$\alpha$) inhibitors; basiliximab (e.g. SIMULECT®); daclizumab (e.g. ZENAPAX®); infliximab (e.g. REMICADE®); mycophenolate mofetil (e.g. CELLCEPT®); azathioprine (e.g. IMURAN®); tacrolimus (e.g. PROGRAF®); steroids; and GI anti-inflammatory agents, for example, sulfasalazine (e.g. AZULFIDINE®); olsalazine (e.g. DIPENTUM®); and mesalamine (e.g. ASACOL®, PENTASA®, ROWASA®).

xvii) Stimulant laxatives, for example, bisacodyl marketed under the trade names DULCOLAX®, FLEET® and EVAC-Q-KWIK®; EX-LAX®; and senna concentrate marketed under the trade names X-PREP® and SENEKOT®.

xviii) Osmotic laxatives, for example, lactulose, PEG, activated charcoal with sorbitol marketed under the trade name ACTIDOSE with SORBITOL®; and phosphate buffered saline.

xix) Absorbents and fiber supplements including fecal softeners, for example, fibers, e.g. bulk fiber laxative plus natural, vegetable stimulant marketed under the trade name PERDIEM®; and bulk forming natural therapeutic fiber, for example, METAMUCIL® and FIBERCON®.

xx) Antacids, such as aluminum and magnesium antacids; and calcium hydroxides such as MAALOX®.

xxi) GI relaxants, for example, cholestyramine resin marketed under the trade name LOCHOLEST® and QUESTRAN®.

xxii) Anti-gas compounds, for example, simethicone marketed under the trade names MYLANTA® and MYLICON®; and enzyme preps including PHAZYME® and BEANO®.

xxiii) Bismuth-containing preparations, for example, bismuth subsalicylate also known as PEPTO-BISMOL®.

xxiv) Pentosan polysulfate, a heparin-like macromolecular carbohydrate derivative which chemically and structurally resembles glycosaminoglycans, marketed under the trade name ELMIRON®.

xxv) Hydroxyzine, for example hydroxyzine HCl, 1-(p-chlorobenzhydryl) 4-[2-(2-hydroxyethoxy)-ethyl] piperizine dihydrochloride, marketed under the trade name ATARAX®.

xxvi) Mast cell stabilizers include e.g. ketotifen marketed under the trade name ZADITEN®.

xxvii) Anti-emetic dopamine $D_2$ antagonists which include e.g. domperidone.

Also included are the pharmaceutically acceptable salts, hydrates, polymorphs, tautomers, racemates, diastereoisomers or enantiomers of all the above-identified first agents or co-agents.

Also within the scope of this invention is the combination of more than two separate active ingredients as set forth above, i.e. a pharmaceutical combination within the scope of this invention could include three active ingredients or more. Further, both the first agent and the co-agent are not the identical active ingredient.

A preferred combination of the invention is one which comprises, as first agent, tegaserod, e.g. in hydrogen maleate salt form, formulated as a solid oral pharmaceutical composition, e.g. a tablet. Representative tegaserod tablets comprise 20 to 60%, e.g. 30 to 50% by weight of a disintegrant; e.g. a super-disintegrant such as crospovidone, based on the total weight of the tablet, e.g. as disclosed in WO 00/10526. An example may be e.g. a tablet comprising 8.31 mg tegaserod in hydrogeno-maleate form (or 6 mg base), 50.00 mg polyplasdone XL, 12.50 mg glyceryl monostearate, 2.50 mg poloxalkol, 37.94 mg lactose, 6.25 mg HPCM, 7.50 mg PEG 4000 and 3.00 mg adsorbed water.

In accordance with another aspect of the invention there is provided a pharmaceutical composition comprising a combination of a first agent and a co-agent as active ingredients, or pharmaceutically acceptable salts, racemates or enantiomers thereof, as defined above under 1.1; 1.2; 1.3 or 1.4, in the presence of a pharmaceutically acceptable carrier, and optionally, other therapeutic ingredients. In a preferred embodiment the first agent according to 1.1 is a 5-$HT_4$ receptor partial agonist, for example, a compound of formula I as defined above having an intrinsic activity <1.00, e.g. a compound of formula I wherein R, is H, Z is —CH= and $R_5$ is OH or $C_{1-6}$alkoxy; in an even more preferred embodiment the first agent is tegaserod, preferably in the hydrogen maleate salt form.

It has surprisingly been found that the pharmaceutical combinations and compositions of the present invention provide an enhanced treatment response for altered gastrointestinal motility, sensitivity and/or secretion and/or abdominal disorders mentioned herein. For example, the combination of tegaserod and PPIs provide not only motility regulation in the upper GI tract but inhibit gastric acid secretion as well which is extremely beneficial for patients suffering from GERD. Furthermore, it has also surprisingly been found that the pharmaceutical combinations and compositions of the present invention provide an enhanced reduction of gastrointestinal pain and other symptoms normally associated with disturbed/altered gastrointestinal motility, sensitivity and/or secretion.

The terms "pharmaceutically acceptable salts" or "a pharmaceutically acceptable salt thereof" refer to salts prepared from pharmaceutically acceptable nontoxic acids or bases including inorganic acids and bases. Suitable pharmaceutically acceptable acid addition salts for the first agent and the co-agents of the present invention include acetic, benzenesulfonic (besylate), benzoic, camphorsulfonic, citric, ethenesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric acid, p-toluenesulfonic, and the like.

To prepare the pharmaceutical compositions of the present invention, the first agent and a co-agent, or their pharmaceutically acceptable salts, racemates or enantiomers are combined in intimate admixture by mixing, blending or combining in any manner known to those of skill in the art, with a pharmaceutically acceptable carrier. The pharmaceutically acceptable carrier may take a wide variety of forms depending on the form of preparation desired for administration.

Any suitable route of administration may be employed for providing a mammal with a therapeutically effective amount of the pharmaceutical combinations and compositions of the present invention. For example, oral, rectal, vaginal, topical, parental (subcutaneous, intramuscular, intravenous, transdermal) and like forms of administration may be employed. Dosage formulations include ointments, foams, gels, transdermal patches, tablets (both fractionable and non-fractionable), caplets, powders for inhalations, gelcaps, capsules , elixirs, syrups, chewable tablets, lozenges, troches, dispersions, aerosols, solutions, fast-dissolving wafers, suppositories or suspensions or other known and effective delivery methods.

In addition to the dosage formulations set out above, the pharmaceutical combinations and compositions of the present invention may also be administered by controlled release means and/or delivery devices such as those described in U.S. Patent Nos. 3,845,770; 3,916,899; 3,536,809; 3,598,123; and 4,008,719 and by "fast-melt" means which include delivery devices which rapidly dissolve in the mouth. Rapid dissolution is meant to include dissolution which takes place in the patient's mouth within less than three minutes. Delivery devices for this type of formulation include; but are not limited to, tablets and capsules. An example of a fast-melt means as used herein is described in U.S. Patent No. 5,178,878 which discloses an effervescent dosage form with microparticles for rapid dissolution of the tablet or capsule.

Oral dosing is preferred. In preparing the compositions in oral dose form, any of the usual pharmaceutical carriers may be employed including any material, composition or vehicle, such as a liquid or solid filler, diluent, excipient, solvent or encapsulating material involved in carrying, formulating or transporting a chemical agent. Specific examples are water, glycols, oils, alcohols and the like in the case of oral liquid preparations. In oral solid forms solid carriers such as starches, sugars, kaolin, lubricants, binders, disintegrating agents and the like may be employed. Oral solid preparations are preferred over the oral liquid preparations. A preferred oral solid preparation is capsules and tablets, because of their ease of administration.

For parental compositions, the carrier will usually comprise sterile water, at least in large part, though other ingredients, to aid solubility for example, may be included. Injectable solutions, for example, may be prepared in which the carrier comprises PEG, saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wetting agent, optionally combined with suitable additives of any nature in minor proportions, which additives do not cause a significant deleterious effect on the skin. It is especially advantageous to formulate the aforementioned pharmaceutical compositions in dosage unit form for ease of administration and uniformity of dosage. Dosage unit form as used herein refers to physically discrete units suitable as unitary dosages, each unit containing a predetermined quantity of active ingredient(s) calculated to produce the desired therapeutic effect in association with the required pharmaceutical carrier.

In another embodiment of the present invention there is provided:

2. A pharmaceutical combination of a first agent and a co-agent, or the pharmaceutically acceptable salts, racemates or enantiomers thereof, or a pharmaceutical composition comprising such a combination in the presence of a pharmaceutically acceptable carrier, as defined above, for use in the treatment of altered gastrointestinal motility, sensitivity and/or secretion and/or abdominal disorders.

3. Use of a pharmaceutical combination of a first agent and a co-agent, or the pharmaceutically acceptable salts, racemates or enantiomers thereof, as defined above, in the manufacture of a medicament for use in the treatment of altered gastrointestinal motility, sensitivity and/or secretion and/or abdominal disorders.

4. Use of a first agent and a co-agent, or the pharmaceutically acceptable salts, racemates or enantiomers thereof, as defined above, in the manufacture of a pharmaceutical composition for use in the treatment of altered gastrointestinal motility, sensitivity and/or secretion and/or abdominal disorders.

5. A method of treating a patient suffering from altered gastrointestinal motility, sensitivity and/or secretion and/or abdominal disorders comprising administering a therapeutically effective amount of a pharmaceutical combination of a first agent and a co-agent, or the pharmaceutically acceptable salts, racemates or enantiomers thereof, or of a pharmaceutical composition comprising such a combination in the presence of a pharmaceutically acceptable carrier, e.g. as defined above, to the patient.

Generally, the pharmaceutical combinations or compositions of the present invention are employed for the treatment of altered gastrointestinal motility, sensitivity and/or secretion and/or abdominal disorders including, but not limited to, heartbum, bloating, postoperative ileus, abdominal pain and discomfort, early satiety, epigastric pain, nausea, vomitting, burbulence, regurgitation, intestinal pseudoobstruction, anal incontinence, GERD, IBS, dyspepsia, chronic constipation or diarrhea, gastroparesis, e.g. diabetic gastroparesis, ulcerative colitis, Crohn's disease, ulcers or the visceral pain associated therewith. In addition, the pharmaceutical combinations and compositions of the invention may also be employed as laxatives, as a preparation for a patient for colonoscopy, or as a means of regulating, stabilizing or normalizing gastrointestinal disorders, through for example, regulation, stabilization or normalization of enterochromaffin cell functions, GI secretion, afferent and efferent fiber activity or abdominal smooth muscle cell activity. The pharmaceutical combinations and compositions of the invention may also be useful in the treatment of menstrual cramps or spastic or interstitial cystitis.

More specifically, the first agent(s) as described above may be combined according to the invention with a co-agent as follows:

Histamine $H_2$ receptor antagonists, PPIs, antacids, bismuth-containing preparations, $GABA_B$ agonists or antagonists to treat GERD, dyspepsia, IBS, visceral pain and other abdominal disorders;

Anxiolytics, SSRIs, tricyclic depressants, somatostatin agonists to treat IBS, dyspepsia, visceral pain, bloating, anal incontinence and other abdominal disorders;

Opioid receptor agonists or antagonists, motilin receptor agonists or antagonists, CRF or CGRP receptor agonists or antagonists, NK or CCK receptor agonists or antagonists, muscarinic M1 receptor agonists or antagonists, antispasmodic agents, NO synthase inhibitors to treat IBS, dyspepsia, visceral pain, bloating, anal incontinence, gastroparesis, chronic constipation, chronic diarrhea, post-operative ileus and other abdominal disorders;

Stimulant laxatives, osmotic laxatives, fecal softeners, absorbents or fiber supplements, GI relaxants, anti-gas compounds to treat chronic constipation, chronic diarrhea, bloating, postoperative ileus and other abdominal disorders.

The therapeutically effective dosage of the pharmaceutical compositions of this invention will vary with the severity of the condition to be treated, and the route of administration. The dose, and perhaps the dose frequency, will also vary according to the age, body weight, and response of the individual patient. In general, the combination of the first agent and the co-agent may be administered in a molar ratio having a range of from about 0.01 to about 2 for the first agent to a range of from about 0.01 to 1000 for the co-agent. As an example, the molar ratio for the first agent to the co-agent is about 1:1000 (first agent to co-agent). As a more specific example, the molar ratio for the first agent to the co-agent may be about 1:1000, 1:500, 1:200, 1:100, 1:20, 1:5, 1:1 or 1:0.01. A preferable molar ratio is about 1:20, even more preferably about 1:5 and most preferably about 1:1.

The total daily dose range, which comprises the above-described molar ratio, for the conditions described herein, may be administered in a range of from about 0.01 mg to about 1000 mg. The daily dose range may be about 800 mg, 600 mg, 400 mg, 200 mg, 100 mg, 50 mg, 20 mg, 10 mg, 5 mg, 1 mg, 0.1 mg or 0.01 mg. Preferably, a daily dose range should be between about 0.5 mg to about 100 mg, while most preferably, a daily dose range should be between about 5 mg to about 75 mg. It is preferred that the doses are administered OD (once daily) or BID (2 times a day). In managing the patient, the therapy should be initiated at a lower dose, perhaps about 5 mg to about 10 mg, and increased up to about 50 mg or higher depending on the patient's response. It may be necessary to use dosages outside these ranges in some cases as will be apparent to those skilled in the art. Further, it is noted that the clinician or treating physician will know how and when to interrupt, adjust, or terminate therapy in conjunction with individual patient response. The term "therapeutically effective amount" is encompassed by the above-described molar ratio and dosage amounts and dose frequency schedule. A "therapeutically effective amount" can be administered in both a fixed or non-fixed combination of a first agent, e.g. tegaserod, and a co-agent.

**EXAMPLES**

The present invention is further described by the following examples. The examples are provided solely to illustrate the invention by reference to specific embodiments. These exemplification's, while illustrating certain specific aspects of the invention, do not portray the limitations or circumscribe the scope of the disclosed invention.

Example 1

Pharmacodynamic effects of tegaserod and co-agents on gastrointestinal and colonic motility

*Animal preparation*: Beagle dogs are used in these experiments. Under halothane anesthesia, four strain-gauge transducers constructed according to Pascaud et al. (Am. J. Physiol.,1978, 235: E532-E538) are sewn on the serosa of the antrum at 5 cm from the pylorus, the duodenum at 10 cm from the pylorus, the jejunum at 50 cm from the ligament of Treitz and the proximal colon at 10 cm from the ileo-colonic junction. Each transducer is sewn with its recording axis parallel to the transverse axis of the gut to measure the contractile force of the circular musde layer. The free ends of the strain-gauge wires are drawn sub-cutaneously to emerge dorsally between the scapulas.

*Recordings*: Calibration of each strain-gauge is performed before implantation. Mechanical activity detected by the transducers is recorded. The motility index of the antrum, duodenum, jejunum and colon is determined according to the technique of Hachet et al. (J. Pharmacol. Meth. (1986) 16: 171-180). The calculated index of motility corresponds to the area between the baseline and the contractile curve during 30 min intervals.

*Study design:* The dogs are separated into groups. Each group receives one of the following regimens: 1) placebo, 2) tegaserod, 3) prucalopride, 4) ADL 8-2698 (LY246736), 5) fluoxetine, 6) tegaserod plus prucalopride, 7) tegaserod + ADL 8-2698, 8) tegaserod + fluoxetine. Compounds at different doses or placebo are administered p.o. to fasted dogs 30 min prior to a meal (water ad libitum). Intravenous infusions of compounds at different doses or placebo (vehicle) to fasted dogs started 30 min prior to a meal (water ad libitum). Gastrointestinal and colonic motility recordings start with the meal intake and are carried out for 6 hours duration in total.

*Data analysis*: Changes in motility index during the 6 hours after the meal associated with the different compounds/administrations are determined at the level of the antrum, duodenum, jejunum and colon.

The combination of tegaserod plus a co-agent significantly increased gastrointestinal and colonic motility as compared to placebo and any of the compounds administered alone.

Example 2

Effects of tegaserod and co-agents on gastric and colonic sensitivity to distension and on the muscular tone of the gut using barostatic distension.

1. Gastric sensitivity and tone

Groups of Wistar rats weighing 200-250 g are used. For surgery, the animals are premedicated with 0.3 ml of acepromazine (0.5 mg/kg) injected intraperitoneally (ip) and anesthetized with 0.3 ml of ketamine injected intraperitoneally.

Animals are positioned in dorsal decubitus and following a xypho-ombilical laparotomy, the stomach is fitted with a permanent balloon connected to a tube introduced in the upper part of the rumen at 1 cm of the gastro-esophageal junction on the great curvature. After closure of the abdomen, rats are positioned in ventral decubitus and one group of 3 stainless steel electrodes (1 m in length - 270 µm in diameter) is implanted into the neck muscles using a technique

described in Ruckebusch and Fioramonti, Gastroenterol. 68:1500-1508,1975. The free ends of electrodes and the catheter of the balloon are exteriorized on the back of the neck and protected by a glass tube attached to the skin. Gastric distension at constant pressure is performed with an electronic barostat (Hachet et al., Gastroenterol Clin Biol, 1993, 17, 347-351). Balloons (5.0-5.5 cm in length) are made with cistern free condoms and sutured to a polyethylene tube (1.0 and 1.8 mm inner and outer diameter respectively, 80 cm in length). The end of the tube is drilled for an easier emptying of the balloon.

Ten days after surgery, electromyographic recordings are performed with an electroencephalograph machine (Reega VIII, Alvar, Paris, France) at a paper speed of 2.4 cm/min. A short time constant of amplification is used to record selectively spike burst (0.03 s). The electromyographic activity is summed every 20 s by an integrator circuit and automatically plotted on a computer.

Under noxious gastric distension, the rat stretches its body and rises up the head and/or turns the head on the left and right sides to observe his flank. The neck muscles are contracted and an electromyographic signal is recorded. In addition, the barostat is connected to a potentiometric recorder for the permanent recording of intragastric pressure. The animals are separated into groups.

After a 30 min period of control recording, the animals receive one of the following regimens: 1) placebo, 2) tegaserod, 3) fedotozine, 4) baclofen, 5) octreotide, 6) fluoxetine, 7) tegaserod + fedotozine, 8) tegaserod + baclofen, 9) tegaserod + octreotide, 10) tegaserod + fluoxetine.

The protocol of gastric distension is started 30 min later.

Electromyographic activity of the neck muscles (EANM) is correlated with changes of posture and is proportional to pain induced by gastric distension. Values integrated every 20 s are summed up for consecutive 10 min. For each stage of distension, neck activity is determined with the following formula :

$$\frac{\text{(EANM at a determined pressure ) - (EANM in basal conditions)}}{\text{EANM in basal conditions}} *100$$

The pain threshold is determined as an increase > 100 % of the electrical activity of the neck muscles.

Gastric volume is determined on the potentiometric recorder as the maximal volume obtained for each stage of distension. Pain threshold and gastric volume are given as mean ± SEM and values compared using Student's "*t*" test for unpaired values.

The pharmaceutical combination of tegaserod and a co-agent significantly decreases the gastric pain associated with gastric distension and increases in gastric tone as compared to placebo and any of the compounds administered alone.

2. Colorectal sensitivity and tone

The influence of tegaserod and a co-agent on rectal or colonic tone and pain is done using barostat distension procedures by applying increasing pressure in a stair-case manner for consecutive periods of 5min.; the volume is measured for each pressure giving an evaluation of the changes in tone.

Wistar rats weighing 220-250 g and housed individually are used. The animals are premedicated with 0.5 mg/kg of acepromazine injected intraperitoneally (IP) and anesthetized by intramuscular administration of 100 mg/kg of ketamine. They are prepared for electromyographic recordings using the technique described in Ruckebusch and

Fioramonti, 1975. Pairs of nichrome wire electrodes (60 cm in length and 80 µm in diameter) are implanted in the striated muscle of the abdomen, 2 cm laterally from the white line. The free ends of electrodes are exteriorized on the back of the neck and protected by a plastic tube attached to the skin.

Electromyographic recordings (time constant : 0.03 sec) started 8 days after surgery. Bipolar recordings of myoelectric activity are performed with an electroencephalographic recorder during one hour starting 30 min before rectal distension.

In order to prevent recording artefacts due to movements during distension, rats are acclimated, 3 days before distension, to stay in tunnel of polypropylene in which distension and EMG recordings are performed. A balloon consisting of a condom (4cm) is introduced into the rectum at 5 cm from the anus and fixed at the base of the tail. The balloon, connected to a barostat, is increasingly inflated with air at pressures of 15, 30, 45 and 60mmHg. each pressure being applied during 5min.

Groups of rats are submitted respectively to the barostatic distension protocol. Ten minutes before they are injected IP with 1) placebo, 2) tegaserod, 3) fedotozine, 4) baclofen, 5) octreotide, 6) fluoxetine, 7) tegaserod + fedotozine, 8) tegaserod + badofen, 9) tegaserod + octreotide, 10) tegaserod + fluoxetine. Statistical analysis of the number of abdominal spike bursts occurring during each 5 min period are performed by Student's "*t*" test fair paired values comparisons after two way ANOVA. P<0.05 is considered statistically significant. Colorectal volumes are given as mean ± SEM and values compared using Student's "*t*" test for unpaired values.

The pharmaceutical combination of tegaserod plus a co-agent significantly decreases the rectal and colonic pain associated with rectal distension and increases colorectal tone as compared to placebo and any of the compounds administered alone.

Example 3

Treatment of non-erosive GERD with the combination of tegaserod and a co-agent

Patients selected for the study are patients with heartburn, the target symptom in patients with non-erosive GERD, as the predominant upper gastrointestinal symptom during the last three (3) months prior to entry into the study and with a history of episodes of heartbum occurring on at least 3 days/week. Patients with GERD and without endoscopic signs of erosive esophagitis are included in the study. Among other factors, patients who are treated with histamine $H_2$-receptor antagonists ($H_2$RAs) in prescription doses or PPIs within one month prior to entry into the baseline phase of the study (Day -14) and patients who need continuous use of PPIs within three months prior to entry into the baseline phase of the study are excluded.

The study consists of a one-week screening period and a 2-week drug-free baseline period, followed by an 8-week, double-blind, placebo-controlled treatment period. During the screening period (Day -21 to Day -14), an endoscopy is performed to rule out the presence of erosive esophagitis. During baseline (Day-14 to Day 1), the patient's symptoms of GERD are documented in a daily diary. At the start of the period, medications for GERD such as $H_2$RAs, PPIs, prokinetics and other disallowed medication are withdrawn and the patients are instructed not to change their diet or lifestyle during the trial. Patients are allowed to take Maalox tablets as a rescue medication for the control of their symptoms. Patients entering the double-blind period have episodes of heartburn on three (3) or more days the last week of the baseline period.

Patients are randomized in equal groups during the double-blind, placebo-controlled period of the study. This period of the study lasts eight (8) weeks and there are 12 treatment arms. The patients in each group receive one of the following regimens: 1) placebo, 2) tegaserod 0.4 mg/day, 3) tegaserod 1 mg/day, 4) tegaserod 4 mg/day, 5) ranitidine 300 mg/day, 6) omeprazole 20 mg/day, 7) tegaserod 0.4 mg/day plus ranitidine 300 mg/day, 8) tegaserod 1 mg/day plus ranitidine 300 mg/day, 9) tegaserod 4 mg/day plus ranitidine 300 mg/day, 10) tegaserod 0.4 mg/day plus omeprazole 20 mg/day, 11) tegaserod 1 mg/day plus omeprazole 20 mg/day and 12) tegaserod 4 mg/day plus omeprazole 20 mg/day, given orally bid for the 8 weeks. The administration as per the twelve (12) groups above is within thirty (30) minutes before meal time in the morning and in the evening. During the 8 weeks, patients continue to complete the daily diary and use only Maalox tablets as rescue medication for the control of their symptoms.

The combination of 1) tegaserod with omeprazole and 2) tegaserod with ranitidine significantly reduces the episodes of heartburn occurring per week during the 8 week period of the double-blind, placebo-controlled period of the study as compared to any of placebo, tegaserod, ranitidine and omeprazole alone. The tegaserod combinations also reduces the other symptoms of GERD including, abdominal pain, bloating and regurgitation. Further, patients show a significant improvement in quality of life factors as compared to any of placebo, tegaserod, ranitidine and omeprazole alone.

A pharmaceutical combination according to the invention, e.g. comprising a 5-$HT_4$ agonist or partial agonist, e.g. tegaserod, and a PPI, e.g. omeprazole, or a histamine $H_2$ receptor antagonist, e.g. ranitidine, or an antacid or a $GABA_B$ receptor agonist or modulator, e.g. baclofen, may also be tested clinically, e.g. using a methodology as disclosed by Talley NJ, et al., in Gastroenterol. Intl., 1993, 6(4), 189:211, or by Veldhuyzen van Zanten SJO et al., in Gut 1999, 45 (Suppl.II), 1169:1177. The dosage is preferably oral and administration may be preferably once or twice a day.

Although the present invention has been described in considerable detail with reference to certain preferred versions thereof, other versions are possible without departing from the spirit and scope of the preferred versions contained herein. All references and Patents (U.S. and others) referred to herein are hereby incorporated by reference in their entirety as if set forth herein in full.

## Claims

**1.** A pharmaceutical combination comprising:

a) a first agent which is a 5-$HT_4$ receptor partial agonist, or a pharmaceutically acceptable salt, racemate or enantiomer thereof; and

b) a co-agent, or a pharmaceutically acceptable salt, racemate or enantiomer thereof.

**2.** A pharmaceutical combination comprising

a) a first agent which is a compound of formula I

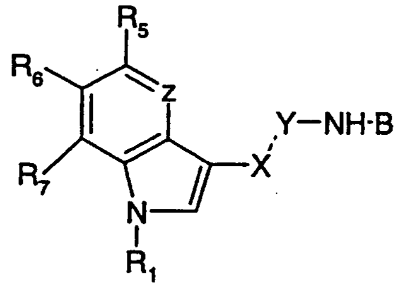 I

wherein

- $R_1$ is hydrogen; $C_{1-6}$alkyl; ($C_{1-6}$alkyl)carbonyl; benzoyl; or phenyl$C_{1-4}$alkyl-carbonyl;
- $R_5$ is hydrogen; halogen; $C_{1-6}$alkyl; hydroxy; nitro; amino; $C_{1-6}$alkylamino; $C_{1-10}$alkylcarbonylamino; $C_{2-6}$alkoxycarbonyl; $SO_2NR_aR_b$ wherein each of $R_a$ and $R_b$ independently is hydrogen or $C_{1-6}$alkyl; cyano; or trimethylsilyl; $C_{1-6}$alkyl substituted by $-SO_2-C_{1-6}$alkyl, $-SO_2NR_aR_b$, $-CONR_aR_b$, $-NH-SO_2-C_{1-6}$alkyl, $-N(C_{1-6}$alkyl$)-SO_2-(C_{1-6}$alkyl), - $NR_aR'_b$ wherein $R'_b$ is hydrogen or $C_{1-6}$alkyl, $C_{2-6}$alkoxycarbonyl or $-PO(C_{1-4}$alkyl$)_2$; carboxy; $CONR_aR_b$; $-PO(C_{1-6}$alkyl$)_2$; $OCONR_cR_d$, wherein each of $R_c$ and $R_d$ independently is $C_{1-6}$alkyl;
- $R_6$ is hydrogen or, when $R_5$ is OH, $R_6$ is hydrogen or halogen,
- Z is $-CR_4=$ wherein $R_4$ is hydrogen, halogen, hydroxy or $C_{1-6}$alkyl or, when $R_5$ is hydrogen or hydroxy, Z is also —N=,
- $R_7$ is hydrogen, halogen, $C_{1-6}$alkyl or $C_{1-6}$alkoxy,
- X—Y is $—CR_8=N-$ or $—CH(R_8)-NH-$ wherein $R_8$ is hydrogen or $C_{1-6}$alkyl, and
- B is a radical of formula (a) or (b),

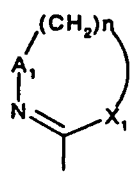

(a)

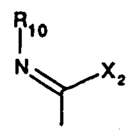

(b)

wherein

- n is 1 or 2,
- $A_1$ is C=O or $CH_2$,
- $X_1$ is S; $NR_{11}$ wherein $R_{11}$ is hydrogen, ($C_{1-6}$alkyl)carbonyl, benzoyl or phenyl$C_{1-4}$alkyl-carbonyl; or $CR_{12}R_{13}$ wherein each of $R_{12}$ and $R_{13}$ independently is hydrogen or $C_{1-4}$alkyl,
- $R_{10}$ is hydrogen; $C_{1-12}$alkyl; $C_{1-6}$alkyl substituted by hydroxy, aryl, aryloxy, adamantyl, a heterocyclic radical, $-NR_{15}-CO-R_{16}$ or $-NH-SO_2$-aryl; $C_{5-7}$cycloalkyl; adamantyl; ($C_{1-10}$alkyl)carbonyl; benzoyl; phenyl ($C_{1-4}$alkyl)carbonyl; or $—CONHR_{14}$,

wherein

- $R_{14}$ is $C_{1-10}$alkyl or $C_{5-7}$cycloalkyl,
- $R_{15}$ is hydrogen or $C_{1-4}$alkyl, and
- $R_{16}$ is $C_{1-6}$alkyl, $C_{5-7}$cycloalkyl, $C_{5-7}$cycloalkyl-$C_{1-4}$alkyl, aryl or aryl$C_{1-4}$alkyl,

wherever "aryl" appears as is or in the significances "aryloxy", "-NH-$SO_2$-aryl" or "aryl($C_{1-4}$alkyl)" in the above definition, it is phenyl or phenyl substituted by halogen, $C_{1-4}$alkyl or $C_{1-6}$alkoxy; and
wherever "heterocyclic radical" appears in the above definition, it is pyridyl, imidazolyl, benzimidazolyl, pyrrolidinyl, pyrrolidonyl, piperidino, pyrazinyl, perhydroindolyl or a radical of formula (c), (d) or (e)

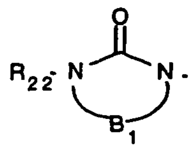

(c)

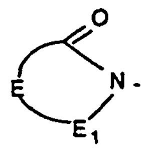

(d)

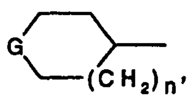

(e)

wherein

$R_{22}$ is hydrogen or $C_{1-4}$alkyl,
$B_1$ is —$CH_2CH_2$-, -$COCH_2$- or —$(CH_2)_3$- in which one or two H thereof can by replaced by $C_{1-4}$alkyl, or 1,2-phenylene,
E is —$CH_2$-$CH_2$-, -$CH_2N(R_{17})$- or —$(CH_2)_3$- in which one or two H thereof can be replaced by $C_{1-6}$alkyl, or 1,2-phenylene,
$E_1$ is CO or $CH_2$,
$R_{17}$ is hydrogen or $C_{1-4}$alkyl,
G is CO, -$CHCOOR_{18}$, -$CHCOR_{19}$, 5,5-dimethyl-1,3-dioxan-2-ylidene or 1,3-dioxolan-2-ylidene, wherein $R_{18}$ is hydrogen or $C_{1-6}$alkyl and $R_{19}$ is $C_{1-6}$alkyl, and
n' is 0 or 1, and
$X_2$ is —$SR_{20}$ or —$NR_3R'_{10}$ wherein $R_{20}$ is $C_{1-6}$alkyl, $R_3$ is hydrogen or $C_{1-6}$alkyl and $R'_{10}$ has one of the significances given for $R_{10}$ above, or $R_3$ and $R'_{10}$ together with the nitrogen atom to which they are attached form a heterocyclic radical as defined above;
with the proviso that where B is a radical of formula (b), only one of $R_{10}$ and $R'_{10}$ can be other than hydrogen and $X_2$ can be —$SR_{20}$ only when $R_{10}$ is hydrogen,
and a physiologically-hydrolysable and -acceptable ether or ester thereof when $R_5$ is hydroxy,
in free form or in pharmaceutically acceptable salt form, and

b) a co-agent, or a pharmaceutically acceptable salt, racemate or enantiomer thereof.

**3.** A pharmaceutical combination according to claim 1 or 2 wherein the co-agent is selected from 5-$HT_3$ receptor antagonists, 5-$HT_4$ receptor agonists or antagonists, compounds which show characteristics of 5-$HT_3$ receptor antagonists and 5-$HT_4$ receptor agonists or antagonists, somatostatin receptor agonists, histamine $H_2$ receptor antagonists, proton pump inhibitors ("PPIs") which include irreversible, reversible and pro-drugs of PPIs, anxiolytics, anti-spasmodic/anti-muscarinic agents, selective serotonin reuptake inhibitors, tricyclic antidepressants, selegeline, $muscarinic_1$ receptor agonists or antagonists, cholecystokinin receptor antagonists, opioid receptor agonists or antagonists, motilin receptor agonists or antagonists, nitric oxide synthase inhibitors, $GABA_B$ receptor agonists or modulators, neurokinin receptor agonists or antagonists, calcitonin gene-related peptide receptor or corticotropin releasing factor receptor agonists or antagonists, anti-inflammatory compounds, stimulant laxatives, osmotic laxatives, fecal softeners, absorbents and fiber supplements, antacids, GI relaxants, anti-gas compounds,

bismuth-containing preparations, pentosan polysulfate, hydroxyzine, mast cell stabilizers and anti-emetic dopamine $D_2$ antagonists.

**4.** A pharmaceutical combination according to claim 1 or 2 wherein the first agent a) is tegaserod, in free form or in pharmaceutically acceptable salt form.

**5.** A pharmaceutical combination comprising

a) a first agent which is selected from the group consisting of $5\text{-}HT_4$ receptor agonists or antagonists and $5\text{-}HT_3$ receptor antagonists, or a pharmaceutically acceptable salt, racemate or enantiomer thereof; and
b) a co-agent which is selected from somatostatin receptor agonists, anxyolitics, benzodiazepine compounds, anti-spasmodic/anti-muscahnic agents, tricyclic antidepressants, selegeline, muscarinic receptor agonists or antagonists, cholecystokinin receptor antagonists, opioid receptor agonists or antagonists, motilin receptor agonists or antagonists, nitric oxide synthase inhibitors, $GABA_B$ receptor agonists or modulators, neurokinin receptor agonists or antagonists, calcitonin gene-related peptide receptor agonists or antagonists, corticotropin releasing factor receptor agonists or antagonists, anti-inflammatory compounds, stimulant laxatives, osmotic laxatives, fecal softeners, absorbents and fiber supplements, antacids, GI relaxants, anti-gas compounds, bismuth-containing preparations, pentosan polysulfate, hydroxyzine, dextromethorphans and mast cell stabilizers, or a pharmaceutically acceptable salt, racemate or enantiomer thereof,

provided that when agent a) is a $5\text{-}HT_3$ receptor antagonist, co-agent b) is other than an antispasmodic/antimuscarinic agent, a muscarinic receptor antagonist, an opioid receptor agonist or antagonist, a neurokinin receptor antagonist, a stimulant laxative, an osmotic laxative or an anti-inflammatory corticosteroid.

**6.** A pharmaceutical combination comprising:

a) a first agent which is a $5\text{-}HT_4$ receptor antagonist or a pharmaceutically acceptable salt, racemate or enantiomer thereof; and
b) a co-agent which is selected from somatostatin receptor agonists, histamine $H_2$ receptor antagonists, PPIs, metoclopramide and anti-emetic dopamine $D_2$ antagonists or a pharmaceutically acceptable salt, racemate or enantiomer thereof.

**7.** A pharmaceutical combination according to claim 1 comprising

a) tegaserod in free form or in pharmaceutically acceptable salt form, and

b) a co-agent which is a proton pump inhibitor or a histamine $H_2$ receptor antagonist.

**8.** A pharmaceutical composition comprising the pharmaceutical combination according to claim 1, 2, 5 or 6 and a pharmaceutically acceptable carrier.

**9.** A pharmaceutical combination according to claim 1, 2, 5 or 6 or a pharmaceutical composition according to claim 8 for use in the treatment of altered gastrointestinal motility, sensitivity and/or secretion and/or abdominal disorders.

**10.** Use of a pharmaceutical combination according to claim 1 or 2 in the manufacture of a medicament for use in the treatment of altered gastrointestinal motility, sensitivity and/or secretion and/or abdominal disorders.

**11.** A method of treating a patient suffering from altered gastrointestinal motility, sensitivity and/or secretion and/or abdominal disorders comprising administering to the patient a therapeutically effective amount of a pharmaceutical combination according to claim 1 or 2, or of a pharmaceutical composition according to claim 8.

European Patent Office

# EUROPEAN SEARCH REPORT

Application Number
EP 04 02 0247

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X<br><br><br><br><br>Y | SCOTT & PERRY: "TEGASEROD" DRUGS,AT,ADIS INTERNATIONAL LTD, vol. 58, no. 3, 1999, pages 492-496, XP000874675 ISSN: 0012-6667<br>* the whole document * | 1,2,4, 8-10<br><br><br><br>3,5,7 | A61K31/4045<br>A61K31/138<br>A61K31/195<br>A61P1/00<br>A61K31/135 |
| X,D<br><br><br>Y | EP 0 505 322 A (SANDOZ AG ;SANDOZ LTD (CH); SANDOZ AG (DE)) 23 September 1992 (1992-09-23)<br>* claims 7,11; example 13 * | 1,2,4, 8-10<br><br>3,5,7 | |
| X | WO 99/17755 A (GLAXO GROUP LTD ;MANGEL ALLEN WAYNE (US); NORTHCUTT ALLISON RUTH () 15 April 1999 (1999-04-15)<br>* abstract * | 5,7-10 | |
| X | WO 94/01095 A (SMITHKLINE BEECHAM PLC ;SANGER GARETH JOHN (GB); BANNER STEPHEN ED) 20 January 1994 (1994-01-20)<br>* abstract * | 5,7-10 | TECHNICAL FIELDS SEARCHED (Int.Cl.7) |
| X | STEADMAN C J ET AL: "Selective 5-Hydroxytryptamine Type 3 Receptor Antagonism With Ondansetron as Treatment for Diarrhea-Predominant Irritable Bowel Syndrome: A Pilot Study" MAYO CLINIC PROCEEDINGS, MAYO MEDICAL VENTURES, ROCHESTER, MN, US, vol. 67, no. 8, August 1992 (1992-08), pages 732-738, XP002098638 ISSN: 0025-6196<br>* abstract * | 5,7-10 | A61K<br>A61P |
| X | WO 93/12785 A (SMITHKLINE BEECHAM PLC) 8 July 1993 (1993-07-08)<br>* abstract * | 5-10 | |

The present search report has been drawn up for all claims

| Place of search | Date of completion of the search | Examiner |
|---|---|---|
| Munich | 27 October 2004 | Herrera, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

Application Number
EP 04 02 0247

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

European Patent Office

## LACK OF UNITY OF INVENTION SHEET B

Application Number
EP 04 02 0247

The Search Division considers that the present European patent application does not comply with the requirements of unity of invention and relates to several inventions or groups of inventions, namely:

1. claims: 1-4 , 5,7-10 (all partially)

   A combination of a 5-HT4 receptor (partial) agonist and a co-agent and use of such a combination in the treatment of certain gastrointestinal disorders

   ---

2. claims: 5-10 (all partially)

   A combination of a 5-HT4 receptor antagonist and a co-agent and use of such a combination in the treatment of certain gastrointestinal disorders

   ---

3. claims: 5,7-10 (all partially)

   A combination of a 5-HT3 receptor antagonist and a co-agent and use of such a combination in the treatment of sertain gastrointestinal disorders

   ---

## ANNEX TO THE EUROPEAN SEARCH REPORT ON EUROPEAN PATENT APPLICATION NO. EP 04 02 0247

This annex lists the patent family members relating to the patent documents cited in the above-mentioned European search report.
The members are as contained in the European Patent Office EDP file on
The European Patent Office is in no way liable for these particulars which are merely given for the purpose of information.

27-10-2004

| Patent document cited in search report | | Publication date | Patent family member(s) | | | Publication date |
|---|---|---|---|---|---|---|
| EP 0505322 | A | 23-09-1992 | AT | 170838 | T | 15-09-1998 |
| | | | AU | 651442 | B2 | 21-07-1994 |
| | | | AU | 1309292 | A | 24-09-1992 |
| | | | BR | 1100237 | A3 | 25-07-2000 |
| | | | CA | 2063671 | A1 | 23-09-1992 |
| | | | CS | 9200858 | A3 | 14-10-1992 |
| | | | DE | 69226894 | D1 | 15-10-1998 |
| | | | DE | 69226894 | T2 | 18-02-1999 |
| | | | DK | 505322 | T3 | 07-06-1999 |
| | | | EP | 0505322 | A1 | 23-09-1992 |
| | | | ES | 2121836 | T3 | 16-12-1998 |
| | | | FI | 921222 | A | 23-09-1992 |
| | | | FI | 971545 | A | 11-04-1997 |
| | | | FI | 20010060 | A | 11-01-2001 |
| | | | HK | 1011028 | A1 | 19-05-2000 |
| | | | HU | 64023 | A2 | 29-11-1993 |
| | | | HU | 9500315 | A3 | 30-10-1995 |
| | | | IE | 920895 | A1 | 23-09-1992 |
| | | | IL | 101312 | A | 18-03-1997 |
| | | | JP | 2593022 | B2 | 19-03-1997 |
| | | | JP | 5086026 | A | 06-04-1993 |
| | | | KR | 198018 | B1 | 15-06-1999 |
| | | | MX | 9201244 | A1 | 01-01-1993 |
| | | | NO | 921104 | A ,B, | 23-09-1992 |
| | | | NZ | 242069 | A | 24-02-1995 |
| | | | RO | 109194 | B1 | 30-12-1994 |
| | | | SG | 43221 | A1 | 17-10-1997 |
| | | | SK | 279214 | B6 | 05-08-1998 |
| | | | RU | 2095347 | C1 | 10-11-1997 |
| | | | US | 5510353 | A | 23-04-1996 |
| | | | ZA | 9202071 | A | 20-09-1993 |
| WO 9917755 | A | 15-04-1999 | AU | 750818 | B2 | 25-07-2002 |
| | | | AU | 9629398 | A | 27-04-1999 |
| | | | BR | 9812886 | A | 08-08-2000 |
| | | | CA | 2305751 | A1 | 15-04-1999 |
| | | | CN | 1281357 | T | 24-01-2001 |
| | | | EA | 3184 | B1 | 27-02-2003 |
| | | | EE | 200000214 | A | 15-06-2001 |
| | | | WO | 9917755 | A2 | 15-04-1999 |
| | | | EP | 1021174 | A2 | 26-07-2000 |
| | | | HR | 20000198 | A1 | 30-04-2001 |
| | | | HU | 0003750 | A2 | 28-10-2001 |
| | | | ID | 23874 | A | 25-05-2000 |
| | | | JP | 2001518495 | T | 16-10-2001 |
| | | | NO | 20001776 | A | 06-06-2000 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 04 02 0247

This annex lists the patent family members relating to the patent documents cited in the above-mentioned European search report.
The members are as contained in the European Patent Office EDP file on
The European Patent Office is in no way liable for these particulars which are merely given for the purpose of information.

27-10-2004

| Patent document cited in search report | Publication date | Patent family member(s) | Publication date |
|---|---|---|---|
| WO 9917755 A | | NZ 503698 A | 25-10-2002 |
| | | PL 340337 A1 | 29-01-2001 |
| | | SK 4862000 A3 | 18-01-2001 |
| | | TR 200000913 T2 | 22-01-2001 |
| | | US 2003036549 A1 | 20-02-2003 |
| | | US 6284770 B1 | 04-09-2001 |
| | | US 2001044450 A1 | 22-11-2001 |
| | | ZA 9809061 A | 05-07-2001 |
| WO 9401095 A | 20-01-1994 | AU 4507693 A | 31-01-1994 |
| | | CA 2139440 A1 | 20-01-1994 |
| | | EP 0650355 A1 | 03-05-1995 |
| | | WO 9401095 A2 | 20-01-1994 |
| | | JP 7508530 T | 21-09-1995 |
| WO 9312785 A | 08-07-1993 | AT 170747 T | 15-09-1998 |
| | | AU 671430 B2 | 29-08-1996 |
| | | AU 3168393 A | 28-07-1993 |
| | | CA 2126354 A1 | 08-07-1993 |
| | | DE 69226961 D1 | 15-10-1998 |
| | | DE 69226961 T2 | 29-04-1999 |
| | | DK 617620 T3 | 07-06-1999 |
| | | EP 0617620 A1 | 05-10-1994 |
| | | ES 2121984 T3 | 16-12-1998 |
| | | WO 9312785 A1 | 08-07-1993 |
| | | JP 7502528 T | 16-03-1995 |
| | | US 2002128172 A1 | 12-09-2002 |

EPO FORM P0459

For more details about this annex : see Official Journal of the European Patent Office, No. 12/82